(19) Europäisches Patentamt
European Patent Office
Office européen des brevets

(11) Veröffentlichungsnummer : **0 107 156**
**B1**

(12) # EUROPÄISCHE PATENTSCHRIFT

(45) Veröffentlichungstag der Patentschrift : **30.12.86**

(21) Anmeldenummer : **83110254.6**

(22) Anmeldetag : **14.10.83**

(51) Int. Cl.[4] : **C 07 D307/81**, C 07 D333/62, C 07 D333/58, C 07 D215/12, C 07 D215/22, C 07 D215/36, C 07 D215/20, C 07 D311/58, C 07 D317/58, C 07 C131/00, C 07 D493/04

(54) **Cyclohexan-1,3-dionderivate, Verfahren zu ihrer Herstellung und ihre Verwendung zur Bekämpfung unerwünschten Pflanzenwuchses.**

(30) Priorität : **22.10.82 DE 3239071**

(43) Veröffentlichungstag der Anmeldung : **02.05.84 Patentblatt 84/18**

(45) Bekanntmachung des Hinweises auf die Patenterteilung : **30.12.86 Patentblatt 86/52**

(84) Benannte Vertragsstaaten : **AT BE CH DE FR GB IT LI NL**

(56) Entgegenhaltungen :
**EP-A- 0 017 195**
**EP-A- 0 046 860**
**CHEMICAL ABSTRACTS, Band 90, Nr. 25, 18. Juni 1979, Seite 576, Nr. 203557t, Columbus, Ohio, US**

(73) Patentinhaber : **BASF Aktiengesellschaft**
**Carl-Bosch-Strasse 38**
**D-6700 Ludwigshafen (DE)**

(72) Erfinder : **Keil, Michael, Dr.**
**Fontanestrasse 4**
**D-6713 Freinsheim (DE)**
Erfinder : **Becker, Rainer, Dr.**
**Im Haseneck 22**
**D-6702 Bad Duerkheim (DE)**
Erfinder : **Goetz, Norbert, Dr.**
**Schoefferstrasse 25**
**D-6520 Worms 1 (DE)**
Erfinder : **Jahn, Dieter, Dr.**
**Burgunder Weg 8**
**D-6803 Edingen-Neckarhausen (DE)**
Erfinder : **Spiegler, Wolfgang, Dr.**
**Westpreussenstrasse 5**
**D-6520 Worms 27 (DE)**
Erfinder : **Wuerzer, Bruno, Dr. Dipl.-Landwirt**
**Ruedigerstrasse 13**
**D-6701 Otterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft Cyclohexan-1,3-dionderivate, Verfahren zu ihrer Herstellung sowie Herbizide, die diese Verbindungen als Wirkstoffe enthalten, und Verfahren zur Bekämpfung unerwünschten Pflanzenwuchses mit diesen Verbindungen.

Es ist bekannt, Cyclohexan-1,3-dionderivate zur selektiven Bekämpfung von unerwünschten Gräsern in breitblättrigen Kulturen zu verwenden (DE-OS 2 439 104).

Aus der EP-A-46 860 sind herbizid wirksame Cyclohexan-1,3-dionderivate bekannt, die in Ringposition 5 einen Cyclohexenyl- oder Cyclohexadienylrest tragen.

Es wurde gefunden, daß Cyclohexan-1,3-dionderivate der Formel

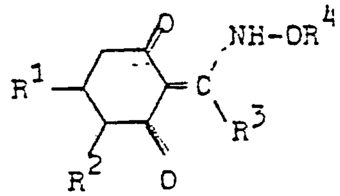

(I)

in der

$R^1$ ein aus zwei aromatischen oder nichtaromatischen Ringen mit jeweils 5 bis 7 Ringgliedern bestehendes kondensiertes Ringsystem, das bis zu drei Heteroatome, ausgewählt aus der Gruppe bestehend aus Sauerstoff, Schwefel und Stickstoff, enthalten und gegebenenfalls durch $C_1$-$C_3$-Alkyl, $C_1$-$C_3$-Alkoxy, $C_1$-$C_3$-Alkylthio oder Halogen substituiert sein kann, mit der Maßgabe, daß mindestens ein Ring ein Heteroatom enthält, wenn beide Ringe nichtaromatisch sind,

$R^2$ Wasserstoff, Methoxycarbonyl, Ethoxycarbonyl, Methyl, Cyano,

$R^3$ $C_1$-$C_4$-Alkyl und

$R^4$ $C_1$-$C_3$-Alkyl, $C_3$-$C_4$-Alkenyl, $C_3$-$C_4$-Halogenalkenyl mit 1 bis 3 Halogensubstituenten oder Propargyl bedeuten,

sowie Salze dieser Verbindungen, gegen Gräser herbizid wirksam und sowohl für breitblättrige Kulturpflanzen als auch monokotyle Kulturen, welche nicht zur Familie der Gräser (Gramineen) zählen, verträglich sind. Überraschenderweise schädigen Verbindungen der Formel I Getreide nicht oder nur wenig.

Die Verbindungen der Formel I können in mehreren isomeren und tautomeren Formen auftreten, die alle vom Patentanspruch umfaßt werden :

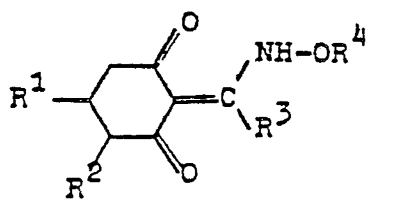

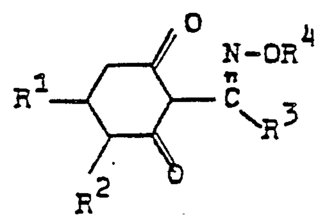

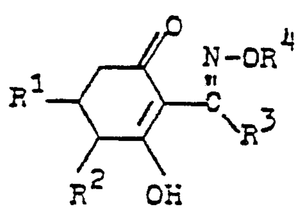

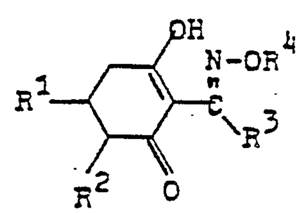

$R^1$ in Formel I bedeutet einaus zwei Ringen mit jeweils 5 bis 7 Ringgliedern bestehendes kondensiertes Ringsystem, das bis zu drei Heteroatome, ausgewählt aus der Gruppe bestehend aus Sauerstoff, Schwefel und Stickstoff, enthalten kann. Ein solches Ringsystem kann sowohl aromatische als auch nichtaromatische Ringe enthalten, mit der Maßgabe, daß mindestens ein Ring ein Heteroatom enthält, wenn beide Ringe nichtaromatisch sind. $R^1$ steht beispielsweise für benzokondensierte oder heteroarylkondensierte, gesättigte oder ungesättigte 5- oder 6-gliedrige heterocyclische Ringe, wie Benzofuryl, Benzothienyl, Indolyl, Benzothiazolyl, Chinolyl, Chinoxalinyl, Chromanyl, 2H-Chromenyl, Benzo-1,3-dioxolyl, Benzo-1,4-dioxenyl, 2,3-Dihydrobenzo [b] furyl, 1,3-Dihydrobenzo [c] furyl, Imidazo

[1,2-a] pyridyl, z. B. 2-Benzo [b] furyl, 3-Benzo [b] furyl, 2-Benzo [b] thienyl, 3-Benzo [b] thienyl, 3-Indolyl, 2-Benzothiazolyl, 3-Chinolyl, 4-Chinolyl, 8-Chinolyl, 2-Chinoxalinyl, 3-Chromanyl, 2H-Chromen-3-yl, Benzo-1,3-dioxol-5-yl, Benzo-1,4-dioxen-6-yl, 2,3-Dihydrobenzo [b] 5-furyl, 3-Imidazo [1,2-a] pyridyl, für aromatische, gegebenenfalls teilweise gesättigte, aus zwei 5- bis 7-gliedrigen Ringen kondensierte Kohlenwasserstoffreste, wie Naphthyl, z. B. 1-Naphthyl, 2-Naphthyl, Azulenyl, z. B. 1-Azulenyl, Tetrahydronaphthyl, z. B. 1,2,3,4-Tetrahydro-1-naphthyl, 1,2,3,4-Tetrahydro-2-naphthyl, Indanyl, z. B. 1-Indanyl, oder für nichtaromatische, aus zwei 5- oder 6-gliedrigen Ringen kondensierte Ringsysteme mit mindestens einem Heteroatom, wie Octahydro-benzo [b]-furyl, Hexahydro-benzo-1,3-dioxolyl, 4a,7,8,8a-Tetrahydro-2H,5H-pyrano [4,3-b] pyranyl, 3,4,4a,7,8,8a-Hexahydro-2H,5H-pyrano [4,3-b] pyranyl, Hexahydrochromanyl, 4a,7,8,8a-Tetrahydro-2H,5H-thiino [3,2-c] pyranyl, 3,4,4a,7,8,8a-Hexahydro-2H,5H-thiino [3,2-c]-pyranyl, 3a,7,8,8a-Tetrahydro-2H,5H-thiino [4,3-b] pyranyl, 3,4,4a,7,8,8a-Hexahydro-2H,5H-thiino [4,3-b] pyranyl, z. B. Octahydro-4-benzo [b] furyl, Hexahydro-benzo-1,3-dioxol-5-yl, 4a,7,8,8a-Tetrahydro-2H,5H-pyrano [4,3-b]-pyran-3-yl, 3,4,4a,7,8,8a-Hexahydro-2H-5H-pyrano [4,3-b] pyran-3-yl, Hexahydrochroman-3-yl, 4a,7,8,8a-Tetrahydro-2H,5H-thiiono [3,2-c] pyran-3-yl, 3,4,4a,7,8,8a-Hexahydro-2H,5H-thiino [3,2-c] pyran-3-yl, 4a,7,8,8a-Tetrahydro-2H,5H-thiino [4,3-b] pyran-3-yl, 3,4,4a,7,8,8a-Hexahydro-2H,5H-thiino [4,3-b] pyran-3-yl.

Diese Reste können durch $C_1$-$C_3$-Alkyl, $C_1$-$C_3$-Alkoxy oder $C_1$-$C_3$-Alkylthio oder durch Halogen substituiert sein. Beispiele für solche substituierten Reste für $R^1$ sind 3-Methyl-2-benzo [b] furyl, 2-Ethyl-3-benzo [b] furyl, 3-Chlor-2-benzo [b] thienyl, 3,6-Dichlor-2-benzo [b] thienyl, 1-Methyl-3-indolyl, 2-Methoxy-3-chinolyl, 2,6-Dimethoxy-3-chinolyl, 2-Ethylthio-3-chinolyl, 2-Chlor-6-methoxy-3-chinolyl, 3,7-Dichlor-8-chinolyl, 2-Methyl-3-chromanyl, 2,2-Dimethyl-3-chromanyl, 2-Methyl-2H-chromen-3-yl, 2,2-Dimethyl-2H-chromen-3-yl, 2-Methyl-3-imidazo [1,2-a] pyridyl, 4,6,8-Trimethyl-1-azulenyl, 6,7-Dimethyl-1,2,3,4-tetrahydro-1-naphthyl, 6-Methoxy-1,2,3,4-tetrahydro-2-naphthyl, 2,2-Dimethyl-cis-octahydro-4-benzo [b] furyl.

$R^3$ in Formel I steht für unverzweigte oder verzweigte Alkylreste mit 1 bis 4 Kohlenstoffatomen, wie Methyl, Ethyl, n-Propyl, i-Propyl, n-Butyl, sec.-Butyl, i-Butyl, tert.-Butyl. Reste für $R^4$ in Formel I sind Propargyl, Alkyl mit 1 bis 3 Kohlenstoffatomen, Alkenyl mit 3 oder 4 Kohlenstoffatomen oder Halogenalkenyl mit 3 oder 4 Kohlenstoffatomen, das bis zu drei Halogensubstituenten enthält, beispielsweise Methyl, Ethyl, n-Propyl, i-propyl, n-Butyl, sec.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,3-Trichlorprop-1-en-3-yl, 1,2-Dibromprop-1-en-3-yl (cis/trans).

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Tetraalkylammoniumsalze, z. B. Tetraethylammoniumsalze, Tetraethylammoniumsalze und Tetrabutylammoniumsalze, Trialkylsulfoniumsalze sowie Trialkylsulfoxoniumsalze, z. B. Trimethylsulfoniumsalze und Trimethylsulfoxoniumsalze, in Betracht.

Bevorzugte Verbindungen der Formel I sind solche, bei denen $R^1$ einen gegebenenfalls durch $C_1$-$C_3$-Alkyl, $C_1$-$C_3$-Alkoxy, $C_1$-$C_3$-Alkylthio oder Halogen substituierten kondensierten Ring, ausgewählt aus der Gruppe bestehend aus Benzo [b] thienyl, Benzo-1,3-dioxolyl, Chinolyl, Tetrahydronaphthyl, 4a,7,8,8a-Tetrahydro-2H,5H-pyrano [4,3-b] pyranyl und 3,4,4a,7,8,8a-Hexahydro-2H,5H-pyrano [4,3-b] pyranyl, beispielsweise 3-Benzo [b] thienyl, Benzo-1,3-dioxol-5-yl, 3-Chinolyl, 8-Chinolyl, 1,2,3,4-Tetrahydro-2-naphthyl, 4a,7,8,8a-Tetrahydro-2H,5H-pyrano [4,3-b] pyran-3-yl und 3,4,4a,7,8,8a-Hexahydro-2H,5H-pyrano [4,3-b] pyran-3-yl bedeutet.

Die Verbindungen der Formel I können durch Umsetzung von 2-Alkanoyl-cyclohexan-1,3-dionen der Formel

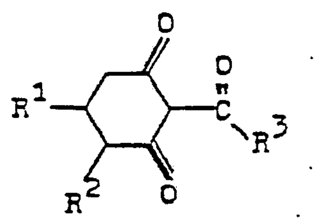

(II)

in der $R^1$, $R^2$ und $R^3$ die obengenannten Bedeutungen haben, mit Hydroxylaminderivaten $R^4O$—$NH_3Y$, in der $R^4$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereichs erfolgt zweckmäßigerweise durch Zusatz von Acetaten, beispielsweise Alkalimetallacetaten, insbesondere von Natrium- oder Kaliumacetat oder einer Mischung aus beiden

Salzen. Alkalimetallacetate werden beispielsweise in Mengen von 0,5 bis 2 Mol, bezogen auf die Ammoniumverbindung der Formel $R^4O{-}NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestilliereu des Lösungsmittels unter vermindertem Druck isoliert werden.

Die Verbindungen der Formel I können außerdem durch Umsetzen der Verbindungen der Formel II mit Hydroxylaminen der Formel $R^4O{-}NH_2$, in der $R_4$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und kaliumalkoholate können als Basen dienen.

Die anderen Metallsalze, z. B. die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Die Tetraalkylammoniumsalze der Verbindungen der Formel I können durch Behandlung dieser Verbindungen mit Tetraalkylammoniumhydroxiden erhalten werden, während die Trialkylsulfonium- bzw. Trialkylsulfoxoniumsalze durch Umsetzung der Natriumsalze mit Trialkylsulfoniumjodid bzw. Trialkylsulfoxoniumjodid erhalten werden können.

Die Verbindungen der Formel II sind neu. Sie können aus Cyclohexan-1,3-dionen der Formel III, die auch in den tautomeren Formeln IIIa und IIIb vorliegen können,

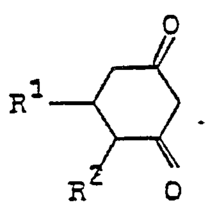

(III)

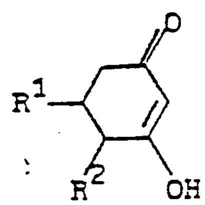

(IIIa)

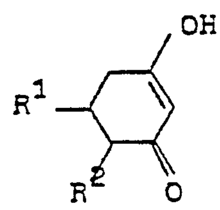

(IIIb)

nach literaturbekannten Methoden (Tetrahedron Letters, *29*, 2491 (1975)) hergestellt werden.

Es ist auch möglich, Verbindungen der Formel II über die Zwischenstufe der Enolester, die bei der Umsetzung von Verbindungen der Formel II eventuell als Isomerengemische anfallen und in Gegenwart von Imidazol- oder Pyridinderivaten umgelagert werden (JP-OS 79/063052), herzustellen.

Zu den Verbindungen der Formel III gelangt man nach literaturbekannten Verfahren, wie dies aus folgendem Schema hervorgeht :

(Siehe Schema Seite 5 f.)

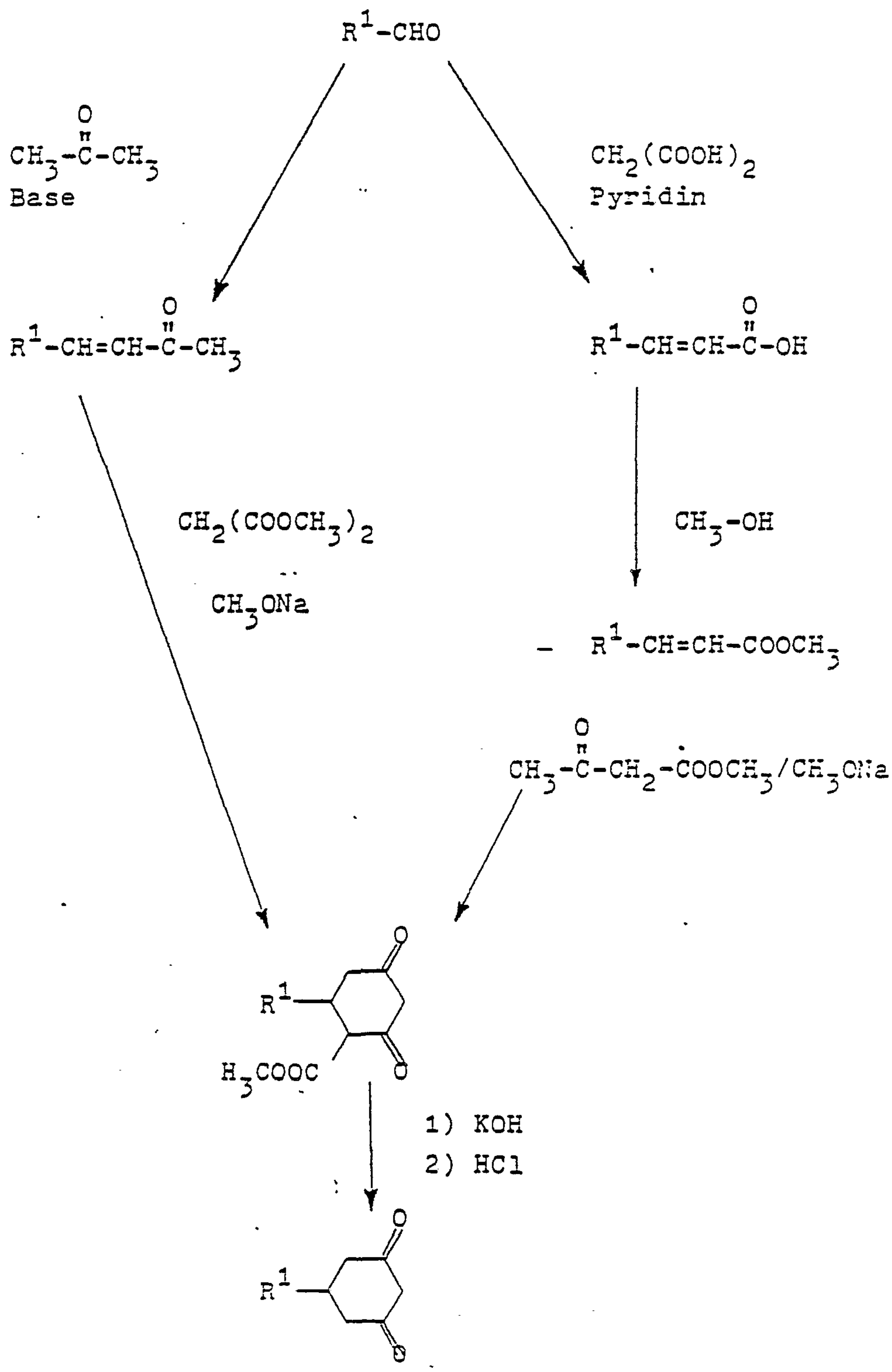


Die Aldehyde der Formel $R^1$—CHO sind durch bekannte Verfahren, wie Vilsmeier-Reaktion, Metallierung und Umsetzung mit Dimethylformamid, Spaltung von Acetalen, Sommelet-Reaktion, Hydroformylierung, Oxidation von primären Alkoholen, Hydrolyse geminaler Dihalogenide, Reduktion von Carbonsäurechloriden, -estern und -nitrilen zugänglich.

Die folgenden Beispiele erläutern die Herstellung der Cyclohexan-1,3-dion-derivate der Formel I. In den Beispielen verhalten sich Gewichtsteile zu Volumenteilen wie Kilogramm zu Liter.

Beispiel 1

4,6 Gew.-Teile 2-Butyryl-5-(2-benzo [b] furyl)-cyclohexan-1, 3-dion, 1,2 Gew.-Teile Allyloxyamin und 30 Vol. Teile Ethanol werden bei Raumtemperatur 12 Stunden gerührt. Das Lösungsmittel wird unter

vermindertem Druck abdestilliert, der Rückstand in 50 Teilen Dichlormethan aufgenommen, die Lösung zweimal mit Wässer gewaschen, über Natriumsulfat getrocknet und das Lösungsmittel unter vermindertem Druck abdestilliert. Man erhält 2-(1-Allyloxyamino-butyliden)-5-(2-benzo [b] furyl)-cyclohexan-1,3-dion in 92 %iger Ausbeute als zähes Öl (Verbindung Nr. 2).

$C_{21}H_{23}NO_4$ (353)
ber. : C 71,4 H 6,6 N 3,9
gef. : C 71,4 H 6,2 N 3,4

Beispiel 2

7,0 Gew.-Teile 2-Butyryl-5-(2-benzo [b] thienyl)-cyclohexan-1,3-dion, 2,4 Gew.-Teile Ethyloxyammoniumchlorid, 2,1 Gew.-Teile Natriumhydrogencarbonat und 70 Teile Methanol werden 12 Stunden bei Raumtemperatur gerührt. Das Lösungsmittel wird unter vermindertem Druck abdestilliert, der Rückstand wird mit 50 Teilen Wasser und 50 Teilen Methylenchlorid extrahiert, die vereinigten organischen Phasen mit Wasser gewaschen, über Natriumsulfat getrocknet und unter vermindertem Druck eingeengt.

Man erhält 2-(1-Ethoxyamino-butyliden)-5-(2-benzo [b] thienyl)-cyclohexan-1,3-dion als hellbraunen Feststoff in 95 %iger Ausbeute vom Fp. 73-76 °C (Verbindung Nr. 10).

$C_{20}H_{23}NO_3S$ (357)
ber. : C 67,2 H 6,4 N 3,9
gef. : C 67,0 H 6,4 N 4,6

Die folgenden Verbindungen der Formel I erhält man durch analoge Umsetzungen :

(Siehe Tabelle Seite 7 ff.)

| Nr. | $R^1$ | $R^2$ | $R^3$ | $R^4$ | $n_D$/Fp [°C] |
|---|---|---|---|---|---|
| 1 | 2-Benzo[b]furyl | H | $n-C_3H_7$ | $C_2H_5$ | |
| 2 | 2-Benzo[b]furyl | H | $n-C_3H_7$ | $CH_2-CH=CH_2$ | |
| 3 | 2-Benzo[b]furyl | H | $C_2H_5$ | $CH_2-CH=CH_2$ | |
| 4 | 2-Ethyl-3-benzo[b]furyl | H | $C_2H_5$ | $C_2H_5$ | 1.5621 (21°C) |
| 5 | 2-Ethyl-3-benzo[b]furyl | H | $C_2H_5$ | $n-C_3H_7$ | |
| 6 | 2-Ethyl-3-benzo[b]furyl | H | $C_2H_5$ | $CH_2-CH=CH_2$ | 1.5695 (21°C) |
| 7 | 2-Ethyl-3-benzo[b]furyl | H | $n-C_3H_7$ | $CH_2-C\equiv CH$ | |
| 8 | 2-Ethyl-3-benzo[b]furyl | H | $n-C_3H_7$ | $CH_2-CCl=CCl_2$ | |
| 9 | 2-Ethyl-3-benzo[b]furyl | $COOCH_3$ | $n-C_3H_7$ | $C_2H_5$ | |
| 10 | 2-Benzo[b]thienyl | H | $n-C_3H_7$ | $C_2H_5$ | 76- 79 |
| 11 | 2-Benzo[b]thienyl | H | $n-C_3H_7$ | $CH_2-CH=CH_2$ | 50 |
| 12 | 2-Benzo[b]thienyl | H | $n-C_3H_7$ | $n-C_3H_7$ | 54 |
| 13 | 2-Benzo[b]thienyl | H | $n-C_3H_7$ | $CH_2-C\equiv CH$ | 72 |
| 14 | 2-Benzo[b]thienyl | H | $n-C_3H_7$ | $CH_2-CH=CHCl$ | 82 |
| 15 | 3-Benzo[b]thienyl | H | $C_2H_5$ | $C_2H_5$ | |
| 16 | 3-Benzo[b]thienyl | H | $C_2H_5$ | $CH_2-CH=CH_2$ | 1.6158 (24°C) |
| 17 | 3-Benzo[b]thienyl | H | $n-C_3H_7$ | $C_2H_5$ | 1.5995 (23°C) |
| 18 | 3-Benzo[b]thienyl | H | $n-C_3H_7$ | $CH_2-CH=CH_2$ | 1.6038 (23°C) |
| 19 | 3-Chlor-2-benzo[b]thienyl | H | $n-C_3H_7$ | $C_2H_5$ | 69- 70 |
| 20 | 3,6-Dichlor-2-benzo[b]thienyl | H | $n-C_3H_7$ | $C_2H_5$ | 106-108 |
| 21 | 3,6-Dichlor-2-benzo[b]thienyl | H | $n-C_3H_7$ | $CH_2-CH=CH_2$ | 71- 74 |
| 22 | 1-Methyl-3-indolyl | H | $n-C_3H_7$ | $CH_2-CH=CH_2$ | |
| 23 | 1-Methyl-3-indolyl | H | $n-C_3H_7$ | $C_2H_5$ | |

(Fortsetzung)

| Nr. | $R^1$ | $R^2$ | $R^3$ | $R^4$ | $n_D$/Fp [°C] |
|---|---|---|---|---|---|
| 24 | 1-Methyl-3-indolyl | H | $C_2H_5$ | $C_2H_5$ | |
| 25 | 1-Methyl-3-indolyl | H | $C_2H_5$ | $CH_2-CH=CH_2$ | |
| 26 | 2-Benzothiazolyl | H | $C_2H_5$ | $CH_2-CH=CH_2$ | |
| 27 | 2-Benzothiazolyl | H | $C_2H_5$ | $C_2H_5$ | |
| 28 | 2-Benzothiazolyl | H | $n-C_3H_7$ | $C_2H_5$ | |
| 29 | 2-Benzothiazolyl | H | $n-C_3H_7$ | $CH_2-CH=CH_2$ | |
| 30 | 3-Chinolyl | H | $n-C_3H_7$ | $C_2H_5$ | 112-115 |
| 31 | 3-Chinolyl | H | $n-C_3H_7$ | $CH_2-CH=CH_2$ | 64 |
| 32 | 2-Methoxy-3-chinolyl | H | $n-C_3H_7$ | $C_2H_5$ | 105-112 |
| 33 | 2-Methoxy-3-chinolyl | H | $n-C_3H_7$ | $CH_2-CH=CH_2$ | 80- 84 |
| 34 | 2-Ethylthio-3-chinolyl | H | $n-C_3H_7$ | $CH_2-CH=CH_2$ | |
| 35 | 2-Ethylthio-3-chinolyl | H | $n-C_3H_7$ | $C_2H_5$ | 103-105 |
| 36 | 2,6-Dimethoxy-3-chinolyl | H | $n-C_3H_7$ | $C_2H_5$ | 114-116 |
| 37 | 2-Chlor-6-methoxy-3-chinolyl | H | $n-C_3H_7$ | $C_2H_5$ | 100-107 |
| 38 | 4-Chinolyl | H | $n-C_3H_7$ | $C_2H_5$ | |
| 39 | 4-Chinolyl | H | $n-C_3H_7$ | $CH_2-CH=CH_2$ | |
| 40 | 3,7-Dichlor-8-chinolyl | H | $n-C_3H_7$ | $CH_2-CH=CH_2$ | |
| 41 | 3,7-Dichlor-8-chinolyl | H | $n-C_3H_7$ | $C_2H_5$ | |
| 42 | 2-Chinoxalinyl | H | $n-C_3H_7$ | $C_2H_5$ | |
| 43 | 2-Chinoxalinyl | H | $n-C_3H_7$ | $CH_2-CH=CH_2$ | |
| 44 | 2-Chinoxalinyl | H | $C_2H_5$ | $CH_2-CH=CH_2$ | |
| 45 | 2-Chinoxalinyl | H | $C_2H_5$ | $C_2H_5$ | |
| 46 | 3-Chromanyl | H | $n-C_3H_7$ | $C_2H_5$ | 88- 92 |

(Fortsetzung)

| Nr. | $R^1$ | $R^2$ | $R^3$ | $R^4$ | $n_D$/Fp [°C] |
|---|---|---|---|---|---|
| 47 | 3-Chromanyl | H | $n-C_3H_7$ | $CH_2-CH=CH_2$ | 67- 71 |
| 48 | 3-Chromanyl | H | $C_2H_5$ | $CH_2-CH=CH_2$ | |
| 49 | 3-Chromanyl | H | $C_2H_5$ | $C_2H_5$ | |
| 50 | 2-Methyl-3-chromanyl | H | $C_2H_5$ | $C_2H_5$ | |
| 51 | 2-Methyl-3-chromanyl | H | $C_2H_5$ | $CH_2-CH=CH_2$ | |
| 52 | 2-Methyl-3-chromanyl | H | $n-C_3H_7$ | $CH_2-CH=CH_2$ | |
| 53 | 2-Methyl-3-chromanyl | H | $n-C_3H_7$ | $C_2H_5$ | |
| 54 | 2,2-Dimethyl-3-chromanyl | H | $n-C_3H_7$ | $C_2H_5$ | |
| 55 | 2,2-Dimethyl-3-chromanyl | H | $n-C_3H_7$ | $CH_2-CH=CH_2$ | |
| 56 | 2,2-Dimethyl-3-chromanyl | H | $C_2H_5$ | $CH_2-CH=CH_2$ | |
| 57 | 2,2-Dimethyl-3-chromanyl | H | $C_2H_5$ | $C_2H_5$ | |
| 58 | 2-Methyl-2H-chromen-3-yl | H | $C_2H_5$ | $C_2H_5$ | 1.5838 (27°C) |
| 59 | 2-Methyl-2H-chromen-3-yl | H | $C_2H_5$ | $CH_2-CH=CH_2$ | 1.5880 (27°C) |
| 60 | 2-Methyl-2H-chromen-3-yl | H | $n-C_3H_7$ | $CH_2-CH=CH_2$ | 1.5830 (22°C) |
| 61 | 2-Methyl-2H-chromen-3-yl | H | $n-C_3H_7$ | $C_2H_5$ | 1.5786 (23°C) |
| 62 | 2,2-Dimethyl-2H-chromen-3-yl | H | $n-C_3H_7$ | $C_2H_5$ | 1.5744 (26°C) |
| 63 | 2,2-Dimethyl-2H-chromen-3-yl | H | $n-C_3H_7$ | $CH_2-CH=CH_2$ | 1.5798 (23°C) |
| 64 | 2,2-Dimethyl-2H-chromen-3-yl | H | $C_2H_5$ | $CH_2-CH=CH_2$ | 1.5804 (26°C) |
| 65 | 2,2-Dimethyl-2H-chromen-3-yl | H | $C_2H_5$ | $C_2H_5$ | 1.5783 (26°C) |
| 66 | Benzo-1,3-dioxol-5-yl | H | $C_2H_5$ | $C_2H_5$ | 1.5638 (23°C) |
| 67 | Benzo-1,3-dioxol-5-yl | H | $C_2H_5$ | $CH_2-CH=CH_2$ | 1.5755 (23°C) |
| 68 | Benzo-1,3-dioxol-5-yl | H | $n-C_3H_7$ | $CH_2-CH=CH_2$ | 67- 68 |
| 69 | Benzo-1,3-dioxol-5-yl | H | $n-C_3H_7$ | $C_2H_5$ | 51- 53 |

(Fortsetzung)

| Nr. | $R^1$ | $R^2$ | $R^3$ | $R^4$ | $n_D$/Fp [°C] |
|---|---|---|---|---|---|
| 70 | 2,3-Dihydro-5-benzo[b]furyl | H | n-$C_3H_7$ | $C_2H_5$ | |
| 71 | 2,3-Dihydro-5-benzo[b]furyl | H | n-$C_3H_7$ | $CH_2$-CH=$CH_2$ | |
| 72 | 2,3-Dihydro-5-benzo[b]furyl | H | $C_2H_5$ | $CH_2$-CH=$CH_2$ | |
| 73 | 2,3-Dihydro-5-benzo[b]furyl | H | $C_2H_5$ | $C_2H_5$ | |
| 74 | 1-Naphthyl | H | n-$C_3H_7$ | $CH_2$-CH=$CH_2$ | 52- 56 |
| 75 | 1-Naphthyl | H | n-$C_3H_7$ | $C_2H_5$ | 86- 88 |
| 76 | 1-Naphthyl | $COOCH_3$ | n-$C_3H_7$ | $C_2H_5$ | 105-110 |
| 77 | 1-Naphthyl | CN | n-$C_3H_7$ | $C_2H_5$ | |
| 78 | 1-Naphthyl | CN | n-$C_3H_7$ | $CH_2$-CH=$CH_2$ | |
| 79 | 1-Naphthyl | CN | $C_2H_5$ | $CH_2$-CH=$CH_2$ | |
| 80 | 1-Naphthyl | CN | $C_2H_5$ | $C_2H_5$ | |
| 81 | 1-Naphthyl | $CH_3$ | n-$C_3H_7$ | $C_2H_5$ | |
| 82 | 1-Naphthyl | $CH_3$ | n-$C_3H_7$ | $CH_2$-CH=$CH_2$ | |
| 83 | 1-Naphthyl | $CH_3$ | n-$C_3H_7$ | n-$C_3H_7$ | |
| 84 | 1-Naphthyl | $CH_3$ | n-$C_3H_7$ | $CH_2$-C≡CH | |
| 85 | 1-Naphthyl | $CH_3$ | n-$C_3H_7$ | $CH_2$-CH=CHCl | |
| 86 | 1-Naphthyl | $CH_3$ | n-$C_3H_7$ | $CH_2$-CCl=$CCl_2$ | |
| 87 | 2-Naphthyl | H | n-$C_3H_7$ | $CH_2$-CH=$CH_2$ | |
| 88 | 2-Naphthyl | H | n-$C_3H_7$ | $C_2H_5$ | |
| 89 | 2-Naphthyl | H | $C_2H_5$ | $CH_2$-CH=$CH_2$ | 1.6015 (23°C) |
| 90 | 2-Naphthyl | H | $C_2H_5$ | $C_2H_5$ | |
| 91 | 2-Naphthyl | $COOCH_3$ | n-$C_3H_7$ | $C_2H_5$ | 105 |
| 92 | 2-Naphthyl | $COOCH_3$ | n-$C_3H_7$ | $CH_2$-CH=$CH_2$ | |

(Fortsetzung)

| Nr. | $R^1$ | $R^2$ | $R^3$ | $R^4$ | $n_D$/Fp [°C] |
|---|---|---|---|---|---|
| 93 | 2-Naphthyl | $COOCH_3$ | $C_2H_5$ | $C_2H_5$ | 52 |
| 94 | 2-Naphthyl | $COOCH_3$ | $C_2H_5$ | $CH_2-CH=CH_2$ | 50- 55 |
| 95 | 1-Azulenyl | H | $n-C_3H_7$ | $C_2H_5$ | |
| 96 | 1-Azulenyl | H | $n-C_3H_7$ | $CH_2-CH=CH_2$ | |
| 97 | 1-Azulenyl | H | $C_2H_5$ | $CH_2-CH=CH_2$ | |
| 98 | 1-Azulenyl | H | $C_2H_5$ | $C_2H_5$ | |
| 99 | 4,6,8-Trimethyl-1-azulenyl | H | $C_2H_5$ | $C_2H_5$ | |
| 100 | 4,6,8-Trimethyl-1-azulenyl | H | $C_2H_5$ | $CH_2-CH=CH_2$ | |
| 101 | 4,6,8-Trimethyl-1-azulenyl | H | $n-C_3H_7$ | $CH_2-CH=CH_2$ | |
| 102 | 4,6,8-Trimethyl-1-azulenyl | H | $n-C_3H_7$ | $C_2H_5$ | |
| 103 | 6,7-Dimethyl-1,2,3,4-tetrahydro--1-naphthyl | H | $n-C_3H_7$ | $C_2H_5$ | |
| 104 | 6,7-Dimethyl-1,2,3,4-tetrahydro--1-naphthyl | H | $n-C_3H_7$ | $CH_2-CH=CH_2$ | |
| 105 | 1,2,3,4-Tetrahydro-2-naphthyl | H | $n-C_3H_7$ | $C_2H_5$ | |
| 106 | 1,2,3,4-Tetrahydro-2-naphthyl | H | $n-C_3H_7$ | $CH_2-CH=CH_2$ | |
| 107 | 1,2,3,4-Tetrahydro-2-naphthyl | H | $C_2H_5$ | $CH_2-CH=CH_2$ | |
| 108 | 1,2,3,4-Tetrahydro-2-naphthyl | H | $C_2H_5$ | $C_2H_5$ | |
| 109 | 6-Methoxy-1,2,3,4-tetrahydro--2-naphthyl | H | $C_2H_5$ | $C_2H_5$ | |
| 110 | 6-Methoxy-1,2,3,4-tetrahydro--2-naphthyl | H | $C_2H_5$ | $CH_2CH=CH_2$ | 104-106 |

(Fortsetzung)

| Nr. | $R^1$ | $R^2$ | $R^3$ | $R^4$ | $n_D$/Fp [°C] |
|---|---|---|---|---|---|
| 111 | 6-Methoxy-1,2,3,4-tetrahydro--2-naphthyl | H | n-$C_3H_7$ | $CH_2CH=CH_2$ | 56- 58 |
| 112 | 6-Methoxy-1,2,3,4-tetrahydro--2-naphthyl | H | n-$C_3H_7$ | $C_2H_5$ | |
| 113 | 1-Indanyl | H | n-$C_3H_7$ | $C_2H_5$ | |
| 114 | 1-Indanyl | H | n-$C_3H_7$ | $CH_2-CH=CH_2$ | |
| 115 | 2,2-Dimethyl-cis-octahydro--4-benzo[b]furyl | H | n-$C_3H_7$ | $CH_2-CH=CH_2$ | |
| 116 | 2,2-Dimethyl-cis-octahydro--4-benzo[b]furyl | H | n-$C_3H_7$ | $C_2H_5$ | |
| 117 | cis-Hexahydro-benzo-1,3--dioxol-5-yl | H | n-$C_3H_7$ | $C_2H_5$ | |
| 118 | cis-Hexahydro-benzo-1,3--dioxol-5-yl | H | n-$C_3H_7$ | $CH_2-CH=CH_2$ | |
| 119 | cis-Hexahydro-benzo-1,3--dioxol-5-yl | H | n-$C_3H_7$ | n-$C_3H_7$ | |
| 120 | cis-Hexahydro-benzo-1,3--dioxol-5-yl | H | n-$C_3H_7$ | $CH_2-C\equiv CH$ | |
| 121 | cis-Hexahydro-benzo-1,3--dioxol-5-yl | H | n-$C_3H_7$ | $CH_2-CH=CHCl$ | |
| 122 | cis-Hexahydro-benzo-1,3--dioxol-5-yl | H | n-$C_3H_7$ | $CH_2-CCl=CCl_2$ | |

(Fortsetzung)

| Nr. | $R^1$ | $R^2$ | $R^3$ | $R^4$ | $n_D$/Fp [°C] |
|---|---|---|---|---|---|
| 123 | 4a,7,8,8a-Tetrahydro-2H,5H--pyrano[4,3-b]pyran-3-yl | H | $n-C_3H_7$ | $C_2H_5$ | 1.5368 (30°C) |
| 124 | 4a,7,8,8a-Tetrahydro-2H,5H--pyrano[4,3-b]pyran-3-yl | H | $n-C_3H_7$ | $CH_2-CH=CH_2$ | 1.5430 (30°C) |
| 125 | 2-Benzo[b]furyl | H | $C_2H_5$ | $C_2H_5$ | |
| 126 | 2-Benzo[b]furyl | H | $C_2H_5$ | $CH_2-CH=CH_2$ | |
| 127 | 3-Methyl-2-benzo[b]furyl | H | $C_2H_5$ | $CH_2-CH=CH_2$ | |
| 128 | 3-Methyl-2-benzo[b]furyl | H | $C_2H_5$ | $C_2H_5$ | |
| 129 | 3-Methyl-2-benzo[b]furyl | H | $n-C_3H_7$ | $C_2H_5$ | |
| 130 | 3-Methyl-2-benzo[b]furyl | H | $n-C_3H_7$ | $CH_2-CH=CH_2$ | |
| 131 | 2-Benzo[b]thienyl | H | $C_2H_5$ | $CH_2-CH=CH_2$ | |
| 132 | 2-Benzo[b]thienyl | H | $C_2H_5$ | $C_2H_5$ | |
| 133 | 3-Chlor-2-benzo[b]thienyl | H | $C_2H_5$ | $C_2H_5$ | |
| 134 | 3-Chlor-2-benzo[b]thienyl | H | $C_2H_5$ | $CH_2-CH=CH_2$ | |
| 135 | 3,6-Dichlor-2-benzo[b]thienyl | H | $C_2H_5$ | $C_2H_5$ | |
| 136 | 3,6-Dichlor-2-benzo[b]thienyl | H | $C_2H_5$ | $CH_2-CH=CH_2$ | |
| 137 | 3-Chinolyl | H | $C_2H_5$ | $CH_2-CH=CH_2$ | |
| 138 | 3-Chinolyl | H | $C_2H_5$ | $C_2H_5$ | |
| 139 | 2-Methoxy-3-chinolyl | H | $C_2H_5$ | $C_2H_5$ | |
| 140 | 2-Methoxy-3-chinolyl | H | $C_2H_5$ | $CH_2-CH=CH_2$ | |
| 141 | 2-Ethylthio-3-chinolyl | H | $C_2H_5$ | $CH_2-CH=CH_2$ | |
| 142 | 2-Ethylthio-3-chinolyl | H | $C_2H_5$ | $C_2H_5$ | |
| 143 | 2,6-Dimethoxy-3-chinolyl | H | $C_2H_5$ | $C_2H_5$ | |

(Fortsetzung)

| Nr. | $R^1$ | $R^2$ | $R^3$ | $R^4$ | $n_D$/Fp [°C] |
|---|---|---|---|---|---|
| 144 | 2,6-Dimethoxy-3-chinolyl | H | $C_2H_5$ | $CH_2-CH=CH_2$ | |
| 145 | 2-Chlor-6-methoxy-3-chinolyl | H | $C_2H_5$ | $CH_2-CH=CH_2$ | |
| 146 | 2-Chlor-6-methoxy-3-chinolyl | H | $C_2H_5$ | $C_2H_5$ | |
| 147 | 4-Chinolyl | H | $C_2H_5$ | $C_2H_5$ | |
| 148 | 4-Chinolyl | H | $C_2H_5$ | $CH_2-CH=CH_2$ | |
| 149 | 3,7-Dichlor-8-chinolyl | H | $C_2H_5$ | $C_2H_5$ | |
| 150 | 3,7-Dichlor-8-chinolyl | H | $C_2H_5$ | $CH_2-CH=CH_2$ | |
| 151 | 1-Naphthyl | H | $C_2H_5$ | $CH_2-CH=CH_2$ | |
| 152 | 1-Naphthyl | H | $C_2H_5$ | $C_2H_5$ | |
| 153 | 6,7-Dimethyl-1,2,3,4-tetrahydro-1-naphthyl | H | $C_2H_5$ | $C_2H_5$ | |
| 154 | 6,7-Dimethyl-1,2,3,4-tetrahydro-1-naphthyl | H | $C_2H_5$ | $CH_2-CH=CH_2$ | |
| 155 | 1-Indanyl | H | $C_2H_5$ | $CH_2-CH=CH_2$ | |
| 156 | 1-Indanyl | H | $C_2H_5$ | $C_2H_5$ | |
| 157 | 2,2-Dimethyl-cis-octahydro-4-benzo[b]furyl | H | $C_2H_5$ | $C_2H_5$ | |
| 158 | 2,2-Dimethyl-cis-octahydro-4-benzo[b]furyl | H | $C_2H_5$ | $CH_2-CH=CH_2$ | |
| 159 | cis-Hexahydro-benzo-1,3-dioxol-5-yl | H | $C_2H_5$ | $CH_2-CH=CH_2$ | |
| 160 | cis-Hexahydro-benzo-1,3-dioxol-5-yl | H | $C_2H_5$ | $C_2H_5$ | |

(Fortsetzung)

| Nr. | $R^1$ | $R^2$ | $R^3$ | $R^4$ | $n_D$/Fp [°C] |
|---|---|---|---|---|---|
| 161 | 4a,7,8,8a-Tetrahydro-2H,5H-pyrano[4,3-b]pyran-3-yl | H | $C_2H_5$ | $C_2H_5$ | |
| 162 | 4a,7,8,8a-Tetrahydro-2H,5H-pyrano[4,3-b]pyran-3-yl | H | $C_2H_5$ | $CH_2-CH=CH_2$ | |
| 163 | 1-Isochinolyl | H | $n-C_3H_7$ | $C_2H_5$ | |
| 164 | 1-Isochinolyl | H | $n-C_3H_7$ | $CH_2-CH=CH_2$ | |
| 165 | 1-Isochinolyl | H | $C_2H_5$ | $CH_2-CH=CH_2$ | |
| 166 | 1-Isochinolyl | H | $C_2H_5$ | $C_2H_5$ | |
| 167 | 4-Isochinolyl | H | $C_2H_5$ | $C_2H_5$ | |
| 168 | 4-Isochinolyl | H | $C_2H_5$ | $CH_2-CH=CH_2$ | |
| 169 | 4-Isochinolyl | H | $n-C_3H_7$ | $CH_2-CH=CH_2$ | |
| 170 | 4-Isochinolyl | H | $n-C_3H_7$ | $C_2H_5$ | |
| 171 | 5-Isochinolyl | H | $n-C_3H_7$ | $C_2H_5$ | |
| 172 | 5-Isochinolyl | H | $n-C_3H_7$ | $CH_2-CH=CH_2$ | |
| 173 | 5-Isochinolyl | H | $C_2H_5$ | $CH_2-CH=CH_2$ | |
| 174 | 5-Isochinolyl | H | $C_2H_5$ | $C_2H_5$ | |
| 175 | 3,4,4a,7,8,8a-Hexahydro-2H,5H-pyrano[4,3-b]pyran-3-yl | H | $n-C_3H_7$ | $C_2H_5$ | 57- 60 |
| 176 | 3,4,4a,7,8,8a-Hexahydro-2H,5H-pyrano[4,3-b]pyran-3-yl | H | $n-C_3H_7$ | $CH_2-CH=CH_2$ | 1,5360 (23°C) |
| 177 | Hexahydrochroman-3-yl | H | $n-C_3H_7$ | $C_2H_5$ | |
| 178 | Hexahydrochroman-3-yl | H | $n-C_3H_7$ | $CH_2-CH=CH_2$ | |
| 179 | 2H-Chromen-3-yl | H | $n-C_3H_7$ | $C_2H_5$ | 50- 56 |

(Fortsetzung)

| Nr. | $R^1$ | $R^2$ | $R^3$ | $R^4$ | $n_D$/Fp [°C] |
|---|---|---|---|---|---|
| 180 | 2H-Chromen-3-yl | H | $n-C_3H_7$ | $CH_2-CH=CH_2$ | 1.5891 (32°C) |
| 181 | 2-Methyl-2H-chromen-3-yl | $COOCH_3$ | $n-C_3H_7$ | $C_2H_5$ | 1.5594 (26°C) |
| 182 | 2-Methyl-2H-chromen-3-yl | $COOCH_3$ | $n-C_3H_7$ | $CH_2-CH=CH_2$ | 1.5623 (26°C) |
| 183 | Benzo-1,3-dioxol-5-yl | H | $n-C_3H_7$ | $CH_2-CBr=CHBr$ | 71 |
| 184 | Benzo-1,3-dioxol-5-yl | H | $C_2H_5$ | $CH_3$ | |
| 185 | Benzo-1,3-dioxol-5-yl | H | $C_2H_5$ | $n-C_3H_7$ | 1.5667 (24°C) |
| 186 | Benzo-1,3-dioxol-5-yl | H | $C_2H_5$ | $CH_2-CH=CHCl$ | 1.5820 (24°C) |
| 187 | Benzo-1,3-dioxol-5-yl | H | $C_2H_5$ | $CH_2-CCl=CCl_2$ | 88- 90 |
| 188 | Benzo-1,3-dioxol-5-yl | H | $C_2H_5$ | $CH_2-C\equiv CH$ | 1.5872 (23°C) |
| 189 | 3-Benzo[b]thienyl | H | $n-C_3H_7$ | $n-C_3H_7$ | 1.5935 (23°C) |
| 190 | Benzo-1,4-dioxen-6-yl | H | $n-C_3H_7$ | $C_2H_5$ | |
| 191 | Benzo-1,4-dioxen-6-yl | H | $n-C_3H_7$ | $CH_2-CH=CH_2$ | |
| 192 | Benzo-1,4-dioxen-6-yl | H | $C_2H_5$ | $n-C_3H_7$ | |
| 193 | Benzo-1,4-dioxen-6-yl | H | $C_2H_5$ | $CH_2-CH=CH_2$ | |
| 194 | Benzo-1,3-dioxol-5-yl | H | $n-C_3H_7$ | $n-C_3H_7$ | 1.5638 (24°C) |
| 195 | 3-Benzo[b]thienyl | H | $n-C_3H_7$ | $n-C_3H_7$ | 1.5935 (23°C) |
| 196 | Hexahydrochroman-3-yl | H | $C_2H_5$ | $C_2H_5$ | |
| 197 | Hexahydrochroman-3-yl | H | $C_2H_5$ | $CH_2-CH=CH_2$ | |
| 198 | 4a,7,8,8a-Tetrahydro-2H,5H--thiino[3,2-c]pyran-3-yl | H | $n-C_3H_7$ | $C_2H_5$ | |
| 199 | 4a,7,8,8a-Tetrahydro-2H,5H--thiino[3,2-c]pyran-3-yl | H | $n-C_3H_7$ | $CH_2-CH=CH_2$ | |

(Fortsetzung)

| Nr. | $R^1$ | $R^2$ | $R^3$ | $R^4$ | $n_D$/Fp [°C] |
|---|---|---|---|---|---|
| 200 | 4a,7,8,8a-Tetrahydro-2H,5H--thiino[3,2-c]pyran-3-yl | H | $C_2H_5$ | $C_2H_5$ | |
| 201 | 4a,7,8,8a-Tetrahydro-2H,5H--thiino[3,2-c]pyran-3-yl | H | $C_2H_5$ | $CH_2-CH=CH_2$ | |
| 202 | 3,4,4a,7,8,8a-Hexahydro-2H,5H--thiino[3,2-c]pyran-3-yl | H | n-$C_3H_7$ | $C_2H_5$ | |
| 203 | 3,4,4a,7,8,8a-Hexahydro-2H,5H--thiino[3,2-c]pyran-3-yl | H | n-$C_3H_7$ | $CH_2-CH=CH_2$ | |
| 204 | 3,4,4a,7,8,8a-Hexahydro-2H,5H--thiino[3,2-c]pyran-3-yl | H | $C_2H_5$ | $C_2H_5$ | |
| 205 | 3,4,4a,7,8,8a-Hexahydro-2H,5H--thiino[3,2-c]pyran-3-yl | H | $C_2H_5$ | $CH_2-CH=CH_2$ | |
| 206 | 4a,7,8,8a-Tetrahydro-2H,5H--thiino[4,3-b]pyran-3-yl | H | n-$C_3H_7$ | $C_2H_5$ | |
| 207 | 4a,7,8,8a-Tetrahydro-2H,5H--thiino[4,3-b]pyran-3-yl | H | n-$C_3H_7$ | $CH_2-CH=CH_2$ | |
| 208 | 4a,7,8,8a-Tetrahydro-2H,5H--thiino[4,3-b]pyran-3-yl | H | $C_2H_5$ | $C_2H_5$ | |
| 209 | 4a,7,8,8a-Tetrahydro-2H,5H--thiino[4,3-b]pyran-3-yl | H | $C_2H_5$ | $CH_2-CH=CH_2$ | |
| 210 | 3,4,4a,7,8,8a-Hexahydro-2H,5H--thiino[4,3-b]pyran-3-yl | H | n-$C_3H_7$ | $C_2H_5$ | |
| 211 | 3,4,4a,7,8,8a-Hexahydro-2H,5H--thiino[4,3-b]pyran-3-yl | H | n-$C_3H_7$ | $CH_2-CH=CH_2$ | |
| 212 | 3,4,4a,7,8,8a-Hexahydro-2H,5H--thiino[4,3-b]pyran-3-yl | H | $C_2H_5$ | $C_2H_5$ | |
| 213 | 3,4,4a,7,8,8a-Hexahydro-2H,5H--thiino[4,3-b]pyran-3-yl | H | $C_2H_5$ | $CH_2-CH=CH_2$ | |
| 214 | Benzo-1,3-dioxol-5-yl (Natriumsalz) | H | $C_2H_5$ | $C_2H_5$ | > 200 |
| 215 | Benzo-1,3-dioxol-5-yl (Tetrabutylammoniumsalz) | H | n-C3H7 | C2H5 | > 200 |

(Fortsetzung)

| Nr. | $R^1$ | $R^2$ | $R^3$ | $R^4$ | $n_D$/Fp [°C] |
|---|---|---|---|---|---|
| 216 | Benzo-1,3-dioxol-5-yl (Bariumsalz) | H | $n-C_3H_7$ | $C_2H_5$ | > 200 |
| 217 | 4a,7,8,8a-Tetrahydro-2H,5H-pyrano[4,3-b]pyran-3-yl | H | $C_2H_5$ | $cis-CH_2CH=CHCl$ | |
| 218 | 4a,7,8,8a-Tetrahydro-2H,5H-pyrano[4,3-b]pyran-3-yl | H | $C_2H_5$ | $trans-CH_2CH=CHCl$ | |
| 219 | 4a,7,8,8a-Tetrahydro-2H,5H-pyrano[4,3-b]pyran-3-yl | H | $n-C_3H_7$ | $cis-CH_2CH=CHCl$ | 1.5543 (22 °C) |
| 220 | 4a,7,8,8a-Tetrahydro-2H,5H-pyrano[4,3-b]pyran-3-yl | H | $n-C_3H_7$ | $trans-CH_2CH=CHCl$ | 1.5542 (22 °C) |
| 221 | 3,4,4a,7,8,8a-Hexahydro-2H,5H,pyrano[4,3-b]pyran-3-yl | H | $C_2H_5$ | $cis-CH_2CH=CHCl$ | |
| 222 | 3,4,4a,7,8,8a-Hexahydro-2H,5H,pyrano[4,3-b]pyran-3-yl | H | $C_2H_5$ | $trans-CH_2CH=CHCl$ | |
| 223 | 3,4,4a,7,8,8a-Hexahydro-2H,5H,pyrano[4,3-b]pyran-3-yl | H | $n-C_3H_7$ | $cis-CH_2CH=CHCl$ | |
| 224 | 3,4,4a,7,8,8a-Hexahydro-2H,5H,pyrano[4,3-b]pyran-3-yl | H | $n-C_3H_7$ | $trans-CH_2CH=CHCl$ | |

$^1$H-NMR-spektroskopische Daten : Chemische Verschiebung in δ-Werten (ppm) in $CDCl_3$, bezogen auf Tetramethylsilan als internen Standard.

Abkürzungen für Signalstrukturen :
s = Singulett
q = Quartlett
d = Dublett
m = Multiplett

| Verbindung Nr. | $-NH-O-CH_2-$ | | |
|---|---|---|---|
| 1 | 4,03 (q) | 6,34 (s) | 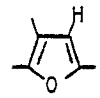 |
| 2 | 4,45 (d) | 6,34 (s) | " |
| 3 | 4,54 (d) | 6,45 (s) | " |
| 15 | 4,15 (d) | 7,15 (s) | 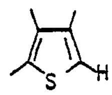 |
| 34 | 4,53 (d) | 3,70 (m) | 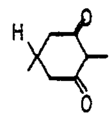 |
| 38 | 4,10 (q) | 4,15 (m) | " |
| 39 | 4,55 (d) | 4,20 (m) | " |
| 40 | 4,50 (d) | 7,50 (s) | 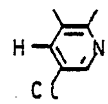 |
| 41 | 4,10 (q) | 7,50 (s) | |
| 70 | 4,02 (q) | 4,45 (t) | 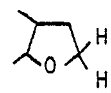 |
| 71 | 4,53 (d) | 4,45 (t) | |
| 87 | 4,53 (d) | 3,50 (m) | 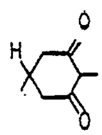 |
| 88 | 4,12 (q) | 3,50 (m) | " |
| 90 | 4,60 (d) | 3,50 (m) | " |
| 92 | 4,53 (d) | 3,55 (s) | $COOCH_3$ |
| 184 | 3,93 (s) $OCH_3$ | 5,97 (s) | $-OCH_2O-$ |

Die Cyclohexan-1,3-dionderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralöfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel

gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkyl-arylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile des Wirkstoffs Nr. 2 mit 10 Gewichtsteilen N-Methyl-α-pyrrolidon und erhält eine Lösung, die zur Anwendung in Form kleinster Tropfen geeignet ist.

II. 10 Gewichtsteile des Wirkstoffs Nr. 10 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile des Wirkstoffs Nr. 3 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile des Wirkstoffs Nr. 38 werden in einer Mischung gelöst, die aus 25 Gewichtsteilen Cyclohexanol, 65 Gewichtsteilen einer Mineralöl-fraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile des Wirkstoffs Nr. 74 werden mit 3 Gewichtsteilen des Natriumsalzes der Diisobutyl-naphthalin-sulfonsäure, 10 Gewichtsteilen des Na-triumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile des Wirkstoffs Nr. 40 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile des Wirkstoffs Nr. 123 werden mit einer Mischung aus 92 Gewichtsteilen pulverförmigem Kieselsäuregel und 8 Gewichtsteilen Paraffinöl, das auf die Oberfläche dieses Kieselsauregels gesprüht wurde, innig vermischt. Man erhält auf diese Weise eine Aufbereitung des Wirkstoffs mit guter Haftfähigkeit.

VIII. 20 Teile des Wirkstoffs Nr. 68 werden mit 2 Teilen Calciumsalz der Dodecylbenzolsulfonsäure, 8 Teilen Fettalkohol-polyglykolether, 2 Teilen Natriumsalze eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Vorzugsweise werden die neuen Wirkstoffe bzw. diese enthaltende Mittel nach dem Auflaufen der unerwünschten Pflanzen ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und

Wachstumsstadien 0,05 bis 5, vorzugsweise 0,1 bis 1,0 kg/ha.

Die Wirkung der Cyclohexan-1,3-dionderivate der Formel I auf das Wachstum von unerwünschten und underwünschten Pflanzen wird durch folgende Gewächshausversuche gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufberhandlung zieht man die Testpflanzen je nach Wuchsform erst bis zu einer Wuchshöhe von 3 bis 15 cm an und behandelt sie danach. Die für die Nachauflaufanwendung benutzten Sojapflanzen werden in einem mit Torfmull (peat) angereicherten Substrat angezogen. Eine Beeinträchtigung der Ergebnisse ist nicht zu befürchten, da es sich um Blattbehandlungen handelt. Ähnliches gilt für den Reis. Zur nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen beispielsweise 0,125, 0,25, 0,5 oder 3,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt, und ihre Reaktion auf die einzelnen Behandlung wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Alopecurus myosuroides (Ackerfuchsschwanz), Avena fatua (Flughafer), Avena sativa (Hafer), Beta vulgaris (Zuckerrübe), Bromus inermis (Unbegrannte Trespe), Echinochloa crus-galli (Hühnerhirse), Glycine max (Sojabohnen), Gossypium hirsutum (Baumwolle), Hordeum vulgare (Gerste), Lolium multiflorum (Ital. Raygras), Oryza sativa (Reis), Panicum spp. (Hirsearten), Poa pratensis (Wiesen-Rispengras), Setaria italica (Kolvenhirse), Sorghum halepense (Aleppohirse), Triticum aestivum (Weizen), Zea mays (Mais).

Vorauflaufanwendung :

Bei Vorauflaufanwendung zeigen beispielsweise die Wirkstoffe Nr. 3, 30, 37, 38, 39, 40, 74, 89, 123 und 124 mit 3,0 kg Wirkstoff/ha eine sehr gute herbizide Wirkung gegen Grasarten. Weiterhin bekämpfen bei dieser Anwendungsmethode beispielsweise die Verbindungen Nr. 12, 13 und 14 unerwünschtes Weidelgras (Ital. raygrass), ohne Kulturhafer zu schädigen.

Nachauflaufanwendung :

Bei der Prüfung im Nachauflaufverfahren zeigen beispielsweise die Verbindungen Nr. 123, 124 und 175 mit 0,125 kg Wirkstoff/ha eine sehr gute Wirkung gegen unerwünschte grasartige Pflanzen, ohne breitblättrige Kulturen, wie Soja und Baumwolle, zu schädigen. Ebenso bekämpfen die Wirkstoffe Nr. 68, 69 und 70 mit 0,25 kg/ha grasartige unerwünschte Pflanzen, ohne breitblättrige Kulturen zu schädigen. Verbindung Nr. 113 bekämpft bei der gleichen Aufwandmenge auch unerwünschte Gräser selektiv in Getreide, die Verbindungen Nr. 41 und Nr. 30 bekämpfen mit 0,5 kg Wirkstoff/ha unerwünschte Gräser in Weisen. Verbindung Nr. 67 hat mit 0,125 kg/ha einebeachtliche herbizide Wirkung gegen das in Reis wichtige Ungras Echinochloa crus-galli mit nur geringen Schädigungen an der Kulturpflanze. Bei einer Aufwandmenge von 3,0 kg/ha zeigen die Verbindungen Nr. 33, 36, 63, 65, 110, 111, 215 und 216 eine hohe Aktivität gegen Grasarten.

In Anbetracht der Verträglichkeit und der Vielseitigkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |

| Botanischer Name | Deutscher Name |
|---|---|
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Fäberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Daucus carota | Möhre |
| Elaeis guineensis | Ölpalme |
| Fragaria vesca | Erdbeere |
| Glycine max | Sojabohne |
| Gossypium hirsutum<br>(Gossypium arboreum<br>Gossypium herbaceum<br>Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus duscis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Spinacia oleracea | Spinat |
| Theobroma cacao | Kakaobaum |

| Botanischer Name | Deutscher Name |
|---|---|
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |

Zur Verbreiterung des Wirkungsspektrums und zur Erzielung synergistischer Effekte können die Cyclohexan-1,3-dionderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und Cyclohexans-1,3-dionderivate anderer Struktur als die der Formel I sowie andere herbizide Wirkstoffe in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Cyclohexan-1,3-dionderivate der Formel

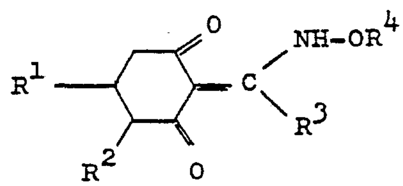

(I)

in der

$R^1$ ein aus zwei aromatischen oder nichtaromatischen Ringen mit jeweils 5 bis 7 Ringgliedern bestehendes kondensiertes Ringsystem, das bis zu drei Heteroatome, ausgewählt aus der Gruppe bestehend aus Sauerstoff, Schwefel und Stickstoff, enthalten und gegebenenfalls durch $C_1$-$C_3$-Alkyl, $C_1$-$C_3$-Alkoxy, $C_1$-$C_3$-Alkylthio oder Halogen substituiert sein kann, mit der Maßgabe, daß mindestens ein Ring ein Heteroatom enthält, wenn beide Ringe nichtaromatisch sind,

$R^2$ Wasserstoff, Methoxycarbonyl, Ethoxycarbonyl, Methyl, Cyano,

$R^3$ $C_1$-$C_4$-Alkyl und

$R^4$ $C_1$-$C_3$-Alkyl, $C_3$-$C_4$-Alkenyl, $C_3$-$C_4$-Halogenalkenyl mit 1 bis 3 Halogensubstituenten oder Propargyl bedeuten,

sowie Salze dieser Verbindungen.

2. Cyclohexan-1,3-dionderivate der Formel I gemäß Anspruch 1, dadurch gekennzeichnet, daß $R^1$ einen gegebenenfalls durch $C_1$-$C_3$-Alkyl, $C_1$-$C_3$-Alkoxy, $C_1$-$C_3$-Alkylthio oder Halogen substituierten kondensierten Ring, ausgewählt aus der Gruppe bestehend aus Benzo [b] thienyl, Benzo-1,3-dioxolyl, Chinolyl, Tetrahydro-naphthyl, 4a,7,8,8a-Tetrahydro-2H,5H-pyrano [4,3-b] pyranyl und 3,4,4a,7,8,8a-Hexahydro-2H,5H-pyrano [4,3-b] pyranyl, bedeutet.

3. 2-(1-Ethoxyamino-butyliden)-5-(4a,7,8,8a-tetrahydro-2H,5H)-pyrano [4,3-b] pyran-3-yl)-cyclohexan-1,3-dion.

4. 2-(1-Ethoxyamino-butyliden)-5-(3,4,4a,7,8,8a-hexahydro-2H,5H-pyrano [4,3-b] pyran-3-yl)-cyclohexan-1,3-dion.

5. 2-(1-Allyloxyamino-butyliden)-5-(3,7-dichlor-8-chinolyl)-cyclohexan-1,3-dion.

6. Verfahren zur Herstellung von Cyclohexan-1,3-dionderivaten der Formel

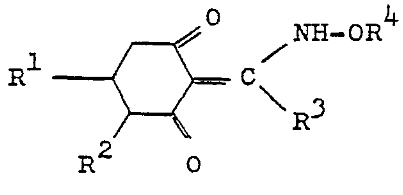

(I)

in der

$R^1$ ein aus zwei aromatischen oder nichtaromatischen Ringen mit jeweils 5 bis 7 Ringgliedern bestehendes kondensiertes Ringsystem, das bis zu drei Heteroatome, ausgewählt aus der Gruppe bestehend aus Sauerstoff, Schwefel und Stickstoff, enthalten und gegebenenfalls durch $C_1$-$C_3$-Alkyl, $C_1$-$C_3$-Alkoxy, $C_1$-$C_3$-Alkylthio oder Halogen substituiert sein kann, mit der Maßgabe, daß mindestens ein Ring ein Heteroatoms enthält, wenn beide Ringe nichtaromatisch sind.

$R^2$ Wasserstoff, Methoxycarbonyl, Ethoxycarbonyl, Methyl, Cyano,

$R^3$ $C_1$-$C_4$-Alkyl und

$R^4$ $C_1$-$C_3$-Alkyl, $C_3$-$C_4$-Alkenyl, $C_3$-$C_4$-Halogenalkenyl mit 1 bis 3 Halogensubstituenten oder Propargyl bedeuten,

dadurch gekennzeichnet, daß man 2-Alkanoylcyclohexan-1,3-dione der Formel

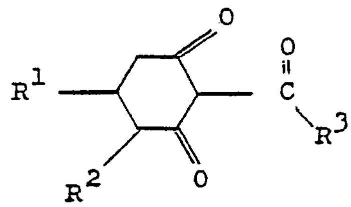
(II)

in der $R^1$, $R^2$ und $R^3$ die oben genannten Bedeutungen haben,

a) mit Ammoniumverbindungen der Formel $R^4O—NH_3Y$, in der $R^4$ die oben 1 genannten Bedeutungen hat und Y ein Anion bedeutet, in einem inerten Verdünnungsmittel gegebenenfalls in Gegenwart von Wasser bei einer Temperatur zwischen 0 und 80 °C in Gegenwart einer Base oder

b) mit einem gegebenenfalls in wäßriger Lösung vorliegenden Hydroxylaminen der Formel $R^4O—NH_2$, in der $R^4$ die oben genannten Bedeutungen hat, in einem inerten Lösungsmittel umsetzt.

7. Herbizid, enthaltend ein Cyclohexan-1,3-dionderivat der Formel I gemäß Anspruch 1.

8. Herbizid, enthaltend inerte Zusatzstoffe und ein Cyclohexan-1,3-dionderivat der Formel I gemäß Anspruch 1.

9. Herbizid, enthaldend inerte Zusatzstoffe und ein Cyclohexan-1,3-dionderivat der Formel I gemäß Anspruch 2.

10. Verfahren zur Bekämpfung unerwünschten Pflanzenwachstums, dadurch gekennzeichnet, daß man die unerwünschten Pflanzen und/oder die von unerwünschtem Pflanzenwachstum freizuhaltende Fläche mit einer herbizid wirksamen Menge eines Cyclohexan-1,3-dionderivates der Formel I gemäß Anspruch 1 behandelt.

11. 2-Alkanoyl-cyclohexan-1,3-dione der Formel

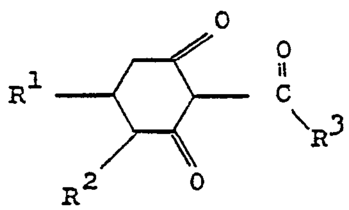
(II)

in der $R^1$, $R^2$ und $R^3$ die im Anspruch 1 genannten Bedeutungen haben.

**Claims**

1. A cyclohexane-1,3-dione derivative of the formula

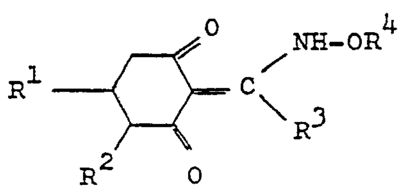
(I)

where

$R^1$ is a fused ring system which consists of two aromatic or non-aromatic rings, each having 5, 6 or 7 ring members, can contain up to three heteroatoms selected from the group consisting of oxygen, sulfur

and nitrogen, and can be unsubstituted or substituted by $C_1$-$C_3$-alkyl, $C_1$-$C_3$-alkoxy, $C_1$-$C_3$-alkylthio or halogen, with the proviso that at least one of the rings contains a heteroatom if both rings are non-aromatic,

$R^2$ is hydrogen, methoxycarbonyl, ethoxycarbonyl, methyl or cyano,

$R^3$ is $C_1$-$C_4$-alkyl, and

$R^4$ is $C_1$-$C_3$-alkyl, $C_3$-$C_4$-alkenyl, $C_3$-$C_4$-haloalkenyl containing 1 to 3 halogen substituents, or propargyl,

or a salt of this compound.

2. A cyclohexane-1,3-dione derivative of the formula I as claimed in claim 1, where $R^1$ is a fused ring which is unsubstituted or substituted by $C_1$-$C_3$-alkyl, $C_1$-$C_3$-alkoxy, $C_1$-$C_3$-alkylthio or halogen, selected from the group consisting of benzo [b] thienyl, benzo-1,3-dioxolyl, quinolyl, tetrahydronaphthyl, 4a,7,8,8a-tetrahydro-2H,5H-pyrano [4,3-b] pyranyl, and 3,4,4a,7,8,8a-hexahydro-2H,5H-pyrano [4,3-b] pyranyl.

3. 2-(1-Ethoxyaminobutylidene)-5-(4a,7,8,8a-tetrahydro-2H,5H-pyrano [4,3-b] pyran-3-yl)-cyclohexane-1,3-dione.

4. 2-(1-Ethoxyaminobutylidene)-5-(3,4,4a,7,8,8a-hexahydro-2H,5H-pyrano [4,3-b] pyran-3-yl)-cyclohexane-1,3-dione.

5. 2-(1-Allyloxyaminobutylidene)-5-(3,7-dichloro-8-quinolyl)-cyclohexane-1,3-dione.

6. A process for the preparation of a cyclohexane-1,3-dione derivative of the formula

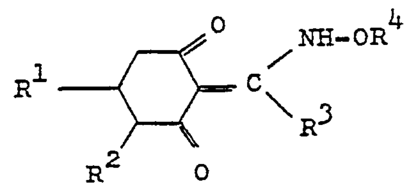

(I)

where

$R^1$ is a fused ring system which consists of two aromatic or non-aromatic rings, each having 5, 6 or 7 ring members, can contain up to three heteroatoms selected from the group consisting of oxygen, sulfur and nitrogen and can be unsubstituted or substituted by $C_1$-$C_3$-alkyl, $C_1$-$C_3$-alkoxy, $C_1$-$C_3$-alkylthio or halogen, with the proviso that at least one of the rings contains a heteroatom if both rings are non-aromatic,

$R^2$ is hydrogen, methoxycarbonyl, ethoxycarbonyl, methyl or cyano,

$R^3$ is $C_1$-$C_4$-alkyl, and

$R^4$ is $C_1$-$C_3$-alkyl, $C_3$-$C_4$-alkenyl, $C_3$-$C_4$-haloalkenyl containing 1 to 3 halogen substituents, or propargyl,

wherein a 2-alkanoylcyclohexane-1,3-dione of the formula

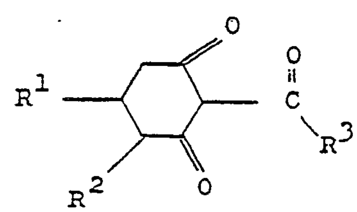

(II)

where $R^1$, $R^2$ and $R^3$ have the above meanings, is reacted

a) with an ammonium compound of the formula $R^4O—NH_3Y$, where $R^4$ has the above meanings and Y is an anion, in an inert diluent in the presence or absence of water at a temperature of from 0 to 80 °C in the presence of a base, or

b) with a hydroxylamine which may be in the form of an aqueous solution and is of the formula $R^4O—NH_2$, where $R^4$ has the above meanings, in an inert solvent.

7. A herbicide containing a cyclohexane-1,3-dione derivative of the formula I, as claimed in claim 1.

8. A herbicide containing inert additives and a cyclohexane-1,3-dione derivative of the formula I, as claimed in claim 1.

9. A herbicide containing inert additives and a cyclohexane-1,3-dione derivative of the formula I, as claimed in claim 2.

10. A process for combating the growth of unwanted plants, wherein the unwanted plants and/or the area to be kept free from unwanted plant growth are treated with a herbicidally effective amount of a cyclohexane-1,3-dione derivative of the formula I as claimed in claim 1.

11. A 2-alkanoylcyclohexane-1,3-dione of the formula

(See formula page 26)

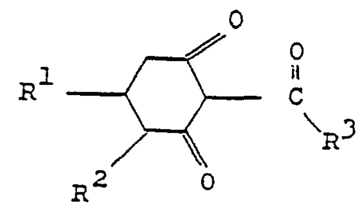

(II)

where $R^1$, $R^2$ and $R^3$ have the meanings given in claim 1.

**Revendications**

1. Dérivés de cyclohexane-1,3-dione de formule

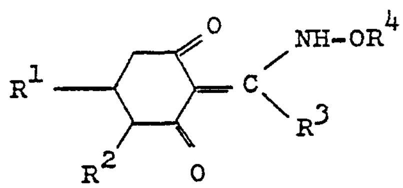

(I)

dans lesquels

$R^1$ représente un système cyclique condensé constitué de deux noyaux aromatiques ou non aromatiques ayant chacun 5 à 7 termes cycliques et qui contient jusqu'à trois hétéroatomes, choisis dans le groupe constitué par oxygène, soufre et azote, et qui peut être substitué éventuellement par alkyle en $C_1$-$C_3$, alcoxy en $C_1$-$C_3$, alkylthio en $C_1$-$C_3$ ou halogène, sous réserve qu'au moins un noyau contienne un hétéroatome, quand deux noyaux sont non aromatiques,

$R^2$ représente hydrogène, méthoxycarbonyle, éthoxycarbonyle, méthyle, cyano,

$R^3$, alkyle en $C_1$-$C_4$ et

$R^4$, alkyle en $C_1$-$C_3$, alcényle en $C_3$-$C_4$, halogénalcényle en $C_3$-$C_4$ avec 1 à 3 substituants halogène ou propargyle,

ainsi que des sels de ces composés.

2. Dérivés de cyclohexane-1,3-dione de formule I, selon la revendication 1, caractérisés par le fait que $R^1$ représente un noyau condensé, éventuellement substitué par alkyle en $C_1$-$C_3$, alcoxy en $C_1$-$C_3$, alkylthio en $C_1$-$C_3$ ou halogène, choisi dans le groupe constitué par benzo [b] thiényle, benzo-1,3-dioxolyle, quinolyle, tétrahydro-naphtyle, 4a,7,8,8a-tétrahydro-2H,5H-pyrano [4,3-b] pyranyle et 3,4,4a,7,8,8a-hexahydro-2H,5H-pyrano [4,3-b] pyranyle.

3. 2-(1-éthoxyamino-butyliden)-5-(4a,7,8,8a-tétrahydro-2H,5H)-pyrano [4,3-b] pyran-3-yl)-cyclohexan-1,3-dione.

4. 2-(1-éthoxyamino-butyliden)-5-(3,4,4a,7,8,8a-hexahydro-2H,5H-pyrano [4,3-b] pyran-3-yl)-cyclohexan-1,3-dione.

5. 2-(1-allyloxyamino-butyliden)-5-(3,7-dichlor-8-quinolyl)-cyclohexan-1,3-dione.

6. Procédé de préparation de dérivés de cyclohexan-1,3-dione, de formule

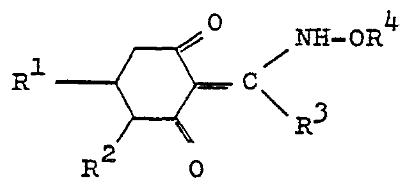

(I)

dans laquelle

$R^1$ représente un système cyclique condensé constitué de deux noyaux aromatiques ou non aromatiques ayant chacun 5 à 7 termes cycliques et qui contient jusqu'à trois hétéroatomes, choisis dans le groupe constitué par oxygène, soufre et azote, et qui peut être substitué éventuellement par alkyle en $C_1$-$C_3$, alcoxy en $C_1$-$C_3$, alkylthio en $C_1$-$C_3$ ou halogène, sous réserve qu'au moins un noyau contienne un hétéroatome, quand deux noyaux sont non aromatiques,

$R^2$ représente hydrogène, méthoxycarbonyle, éthoxycarbonyle, méthyle, cyano,

$R^3$ alkyle en $C_1$-$C_4$ et

$R^4$ alkyle en $C_1$-$C_3$, alcényle en $C_3$-$C_4$, halogénalcényle en $C_3$-$C_4$ avec 1 à 3 substituants halogène ou propargyle

caractérisé par le fait que l'on fait réagir des 2-alcanoyl-cyclohexane-1,3-dione de formule

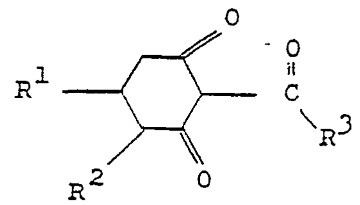

(II)

dans laquelle $R^1$, $R^2$ et $R^3$ ont les significations indiquées plus haut,

a) avec des composés d'ammonium de formule $R^4O—NH_3Y$, dans laquelle $R^4$ a les significations indiquées plus haut et Y représente un anion, dans un diluant inerte, éventuellement en présence d'eau, à une température comprise en 0 et 80 °C, en présence d'une base, ou

b) dans un solvant inerte avec une hydroxylamine de formule $R^4O—NH_2$, se trouvant éventuellement en solution aqueuse, où $R^4$ a les significations indiquées plus haut.

7. Herbicide, contenant un dérivé de cyclohexane-1,3-dione de formule 1 selon la revendication 1.

8. Herbicide, contenant des additifs inertes et un dérivé de cyclohexane-1,3-dione selon la revendication 1.

9. Herbicide, contenant des additifs inertes et un dérivé de cyclohexane-1,3-dione de formule I selon la revendication 2.

10. Procédé de lutte contre la croissance des plantes indésirables, caractérisé par le fait que l'on traite les plantes indésirables et/ou les surfaces à maintenir débarassées d'une croissance de plantes indésirables avec une quantité activé au point de vue herbicide d'un dérivé de cyclohexane-1,3-dione de formule I selon la revendication 1.

11. 2-alcanoyl-cyclohexane-1,3-dione de formule

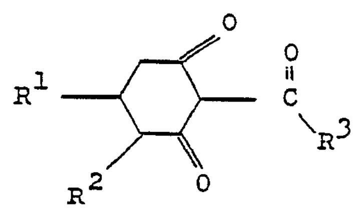

(II)

dans laquelle $R^1$, $R^2$ et $R^3$ ont les significations indiquées dans la revendication 1.